(19) Europäisches Patentamt
European Patent Office
Office européen des brevets

(11) **EP 3 143 060 B1**

(12) **EUROPEAN PATENT SPECIFICATION**

(45) Date of publication and mention of the grant of the patent:
**24.06.2020 Bulletin 2020/26**

(21) Application number: **15723462.6**

(22) Date of filing: **07.05.2015**

(51) Int Cl.:
***C08G 18/00*** *(2006.01)* ***C08G 18/16*** *(2006.01)*
***C08G 18/22*** *(2006.01)*

(86) International application number:
**PCT/EP2015/060062**

(87) International publication number:
**WO 2015/173111 (19.11.2015 Gazette 2015/46)**

(54) **CATALYSTS FOR THE SYNTHESIS OF OXAZOLIDINONE COMPOUNDS**
KATALYSATOREN FÜR DIE SYNTHESE VON OXAZOLIDINONVERBINDUNGEN
CATALYSEURS POUR LA SYNTHÈSE DE COMPOSÉS D'OXAZOLIDINONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2014 EP 14167823**
**13.02.2015 EP 15154978**

(43) Date of publication of application:
**22.03.2017 Bulletin 2017/12**

(73) Proprietor: **Covestro Deutschland AG**
**51373 Leverkusen (DE)**

(72) Inventors:
- **MÜLLER, Thomas Ernst**
**52074 Aachen (DE)**
- **GÜRTLER, Christoph**
**50735 Köln (DE)**
- **BASU, Susmit**
**52070 Aachen (DE)**
- **LATORRE, Irene**
**51373 Leverkusen (DE)**
- **RANGHEARD, Claudine**
**NL-6211 CB Maastricht (NL)**
- **LEITNER, Walter**
**52074 Aachen (DE)**

(74) Representative: **Levpat**
**c/o Covestro AG**
**Gebäude 4825**
**51365 Leverkusen (DE)**

(56) References cited:
**EP-A1- 0 296 450** **EP-A2- 0 342 064**
**WO-A1-86/06734** **US-A- 3 687 897**

EP 3 143 060 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a method for the selective production of oxazolidinone compounds comprising the step of reacting an isocyanate compound with an epoxide compound in the presence of an onium salt as catalyst and to the oxazolidinone compounds obtainable by said method. The invention further relates to a method for the production of oligooxazolidinone and/or polyoxazolidinone compounds, comprising the step of reacting a polyisocyanate compound with a polyepoxide compound in the presence of said catalyst. The invention further relates to oxazolidinone, oligooxazolidinone and/or polyoxazolidinone compounds with a regioselectivity to the 5-substituted 1,3-oxazolidin-2-one regioisomer of ≥ 78 %.

Oxazolidinones are widely used structural motifs in pharmaceutical applications, and the cycloaddition of epoxides and isocyanates seems to be a convenient one-pot synthetic route to produce them. Expensive catalysts, reactive polar solvents, long reaction times and low chemoselectivities are common in early reports for the synthesis of oxazolidinones (M. E. Dyen and D. Swern, Chem. Rev., 67, 197 (1967); X. Zhang and W. Chen, Chem. Lett., 39, 527 (2010); M.T. Barros and A.M.F. Phillips, Tetrahedron: Asymmetry, 21, 2746 (2010); H.-Y. Wu, J.-C. Ding and Y.-K. Liu, J. Indian Chem. Soc., 80, 36 (2003); C. Qian and D. Zhu, Synlett, 129 (1994)). An alternative access to the formation of oxazolidinones, which allows for a high reaction rate and enables to obtain the oxazolidinone moiety with high chemoselectivity and high regioselectivity to the 5-substituted 1,3-oxazolidin-2-one regioisomer, was required.

Unpublished European Patent Application No. 12192611.7 relates to a method for the production of oxazolidinone compounds, comprising the step of reacting an isocyanate compound with an epoxide compound in the presence of a Lewis acid catalyst. The use of onium catalysts is disclosed. But neither activities nor selectivities with regard to the substitution pattern of the onium salts have been disclosed. Especially an antimony catalyst ($Ph_4SbBr$) was used as Lewis acidic catalyst, which has certain toxicity.

EP 0296450 discloses a method for the production of oligomeric oxazolidinone containing polyepoxides from bisepoxides and diisocyanates. It is required that the used bisepoxides carry hydroxy-groups corresponding to an OH-number of at least 2 and the molar ratio between the epoxide and isocyanate group within the diepoxides and diisocyanates is 1.4 up to 2.5. The reaction is carried out at temperatures between 140°C and 180°C. The catalysts claimed are alkyl- and aryl-substituted phosphonium salts; in the examples tetrabutylphosphonium bromide and benzyltriphenylphosphonium bromide are used. A high selectivity towards oxazolidinone and suppression of the trimerization reaction are mentioned. The regioselectivity is not discussed and the chemoselectivity is induced by hydroxyl groups within the diepoxide.

EP 0343064 discloses a method for preparing a thermosettable resin from polyisocyanate, polyepoxide and catalyst. The catalysts used are tin compounds as well as stibonium and phosphonium salts. A high selectivity to oxazolidinone in comparison to isocyanurate is observed. The regioselectivity is not discussed.

US 3687897 discloses a process for the preparation of oxazolidinones by reacting an isocyanate with an epoxide in presence of a phosphonium catalyst such as tetrabutylphosphonium bromide. The examples disclose the use of a tetrabutylphosphonium bromide or iodide catalyst. The influence of the substituents on the phosphonium salt has not been discussed. Phosphonium salts having three phenyl substituents at the phosphorous atom are deemed inactive with regard to the reaction of isocyanate and epoxide. The regio- and chemoselectivity of the reaction is not discussed.

WO 86/06734 A1 discloses a process for the preparation of polyisocyanurate-based polyoxazolidinone polymers containing relatively small proportions of trimerized polyisocyanates. The amount of isocyanurate compounds is below 15 mol-%. The catalysts used are organoantimony iodide salts. The regioselectivity of the reaction is not discussed.

It was therefore desirable to provide a catalyst system for the production of oxazolidinones as well as of oligomeric and polymeric oxazolidinone compounds by reacting an isocyanate compound with an epoxide compound, which enables short reaction times and provides the oxazolidinone compound with high chemoselectivity and with a regioselectivity towards the 5-substituted 1,3-oxazolidin-2-one regioisomer of ≥ 78 %. In the manufacture of polyoxazolidinones by reacting diisocyanates with diepoxides in the presence of the catalyst system according to the invention, certain side reactions should be avoided to obtain polymers with useful properties. Such undesired side reactions include for example the trimerisation of isocyanate to isocyanurate groups, the formation of carbodiimides and homo-polymers of the epoxides. The specifications concerning a high chemoselectivity to the oxazolidinone compound are particularly stringent, if polyoxazolidinone compounds with an almost linear polymer backbone and thermoplastic properties are desired. In particular, the formation of isocyanurate groups should be supressed as this side reaction may result in crosslinking of the polymer chains.

According to the present invention this was achieved by the use of particular onium salts as catalyst. The use of such special onium salts leads to a high rate in the reaction of isocyanates and epoxides and enables the production of the oxazolidinone compounds with high chemoselectivity in combination with the desired regioselectivity towards the 5-substituted 1,3-oxazolidin-2-one regioisomer of ≥ 78 %.

Surprisingly, it has been found that the substitution pattern of the onium cation determines the activity and selectivity of the catalysts.

Therefore the subject matter of the present invention is a method for the production of oxazolidinone compounds, comprising the step of reacting an isocyanate compound with an epoxide compound in the presence of a catalyst, characterized in that the catalyst is represented by the general formula (I)

$$[M(R1)(R2)(R3)(R4)]^{+}{}_{n}Y^{n-} \qquad (I)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with one or more alkyl groups containing 1 to 10 carbon atoms and/or heteroatom containing substituents and/or heteroatoms,
whereas
(R4) is different from (R1), (R2), and (R3) and
wherein (R4) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and aryl groups, containing 6 carbon atoms which are substituted with a heteroatom containing group,
whereas
(R1), (R2) are aryl groups containing 6 to 18 carbon atoms preferably 6 carbon atoms,
Y is a halide, carbonate, nitrate, sulphate or phosphate anion and
n is an integer of 1, 2 or 3.

As used herein, the term "oxazolidinone compound" is meant to denote oxazolidinone compounds obtainable by the reaction of an isocyanate compound with an epoxide compound. The term "oxazolidinone compound" is meant to include polyoxazolidinone compounds with at least two oxazolidinone moieties in the molecule obtainable by the reaction of a polyisocyanate with a polyepoxide.

As used herein, the term "isocyanate compound" is meant to denote monoisocyanate compounds, polyisocyanate compounds (having two or more NCO groups), NCO-terminated biuret, isocyanurates, uretdiones, carbamates and NCO-terminated prepolymers. The term "monoisocyanate compound" is meant to denote isocyanate compounds having one isocyanate group. The term "polyisocyanate compound" is meant to denote isocyanate compounds having at least two isocyanate groups. The term "diisocyanate compound" is meant to denote polyisocyanate compounds having two isocyanate groups.

As used herein, the term "epoxide compound" is meant to denote monoepoxide compounds, polyepoxide compounds (having two or more epoxide groups) and epoxide terminated prepolymers. The term "monoepoxide compound" is meant to denote epoxide compounds having one epoxy group. The term "polyepoxide compound" is meant to denote epoxide compounds having at least two epoxy groups. The term "diepoxide compound" is meant to denote epoxide compounds having two epoxy groups.

As used herein, the term "onium salt" is meant to denote the combination of a particular phosphonium and stibonium cation with a suitably chosen anion.

Examples of suitable monoisocyanate compounds are methyl isocyanate, ethyl isocyanate, n-propyl isocyanate, isopropyl isocyanate, n-butyl isocyanate, isobutyl isocyanate, tert-butyl isocyanate, n-pentyl isocyanate, n-hexyl isocyanate, cyclohexyl isocyanate, ω-chlorohexamethylene isocyanate, n-heptyl isocyanate, n-octyl isocyanate, iso-octyl isocyanate, 2-ethyl hexyl isocyanate, 2-norbornyl methyl isocyanate, nonyl isocyanate, 2,3,4-trimethylcyclohexyl isocyanate, 3,3,5-trimethylcyclohexyl isocyanate, decyl isocyanate, undecyl isocyanate, dodecyl isocyanate, tridecyl isocyanate, tetradecyl isocyanate, pentadecyl isocyanate, hexadecyl isocyanate, octadecyl isocyanate, stearyl isocyanate, 3-butoxypropyl isocyanate, 3-(2-ethylhexyloxy)-propyl isocyanate, 6-chlorohexyl isocyanate benzyl isocyanate, phenyl isocyanate, *ortho-, meta-, para-tolyl* isocyanate, dimethylphenyl isocyanate (technical mixture and individual isomers), 4-pentylphenyl isocyanate, 4-cyclohexylphenyl isocyanate, 4-dodecylphenyl isocyanate, *ortho-, meta-, para*-methoxyphenyl isocyanate, chlorophenyl isocyanate (2,3,4-isomers), the different dichlorophenyl isocyanate isomers, 4-nitrophenyl isocyanate, 3-trifluoromethylphenyl isocyanate, 1-naphthyl isocyanate.

Preferred monoisocyanate compounds are benzyl isocyanate, phenyl isocyanate, *ortho-, meta-, para-tolyl* isocyanate, dimethylphenyl isocyanate (technical mixture and individual isomers), 4-cyclohexylphenyl isocyanate and *ortho-, meta-, para*-methoxyphenyl isocyanate.

A mixture of two or more of the aforementioned monoisocyanate compounds can also be used.

Examples of polyisocyanate compounds are tetramethylene diisocyanate, hexamethylene diisocyanate (HDI), 2-methylpentamethylene diisocyanate, 2,2,4-trimethylhexamethylene diisocyanate (THDI), dodecamethylene diisocyanate, 1,4-diisocyanatocyclohexane, 3-isocyanatomethyl-3,3,5-trimethylcyclohexyl isocyanate (isophorone diisocyanate, IPDI), 4,4'-diisocyanatodicyclohexylmethane ($H_{12}$-MDI), 4,4'-diisocyanato-3,3'-dimethyldicyclohexylmethane, 4,4'-diisocyanato-2,2-dicyclohexylpropane, poly(hexamethylene diisocyanate), octamethylene diisocyanate, tolylene-$\alpha$,4-diisocyanate, poly(propylene glycol) tolylene-2,4-diisocyanate terminated, poly(ethylene adipate) tolylene-2,4-diisocyanate terminated, 2,4,6-trimethyl-1,3-phenylene diisocyanate, 4-chloro-6-methyl-1,3-phenylene diisocyanate, poly[1,4-phenylene diisocyanate-co-poly(1,4-butanediol)] diisocyanate, poly(tetrafluoroethylene oxide-co-difluoromethylene oxide) $\alpha,\omega$-diisocyanate, 1,4-diisocyanatobutane, 1,8-diisocyanatooctane, 1,3-bis(1-isocyanato-1 -methylethyl)benzene, 3,3' -dimethyl-4,4' -biphenylene diisocyanate, naphthalene-1,5-diisocyanate, 1,3-phenylene diisocyanate, 1,4-diisocyanatobenzene, 2,4- or 2,5- or 2,6-diisocyanatotoluene (TDI) or mixtures of these isomers, 4,4'-, 2,4'- or 2,2'-diisocyanatodiphenylmethane (MDI) or mixtures of these isomers, 4,4'-, 2,4'- or 2,2'-diisocyanato-2,2-diphenylpropane-p-xylene diisocyanate and $\alpha,\alpha,\alpha',\alpha'$-tetramethyl- m- or -p-xylene diisocyanate (TMXDI), mixtures thereof or biurets, isocyanurates, carbamates or uretdiones of the aforementioned isocyanates.

Preferred polyisocyanates are hexamethylene diisocyanate (HDI), 3-isocyanatomethyl-3,3,5-trimethylcyclohexyl isocyanate (isophorone diisocyanate, IPDI), 4,4'-diisocyanatodicyclohexylmethane ($H_{12}$-MDI), 2,4- or 2,5- or 2,6-diisocyanatotoluene (TDI) or mixtures of these isomers, 4,4'-, 2,4'- or 2,2'-diisocyanatodiphenylmethane (MDI) or mixtures of these isomers.

A mixture of two or more of the aforementioned polyisocyanates can also be used.

Examples of monoepoxide compounds are ethylene oxide, propylene oxide, 1,2-butene oxide, 2,3-butene oxide, butadiene monoepoxide, 1,2-hexene oxide, cyclohexene oxide, vinylcyclohexene monoxide, limonene monoxide, oxides of C10 - C18 alpha-olefins, styrene oxide, the epoxides of unsaturated fatty acid C1 - C18 alkyl esters, methyl glycidyl ether, ethyl glycidyl ether, propyl glycidyl ether, butyl glycidyl ether, pentyl glycidyl ether, hexyl glycidyl ether, cyclohexyl glycidyl ether, octyl glycidyl ether, 2-ethylhexyl glycidyl ether, C10 - C18 alkyl glycidyl ether, allyl glycidyl ether, benzyl glycidyl ether, phenyl glycidyl ether, 4-tert-butylphenyl glycidyl ether, 1-naphthyl glycidyl ether, 2-naphthyl glycidyl ether, 2-chlorophenyl glycidyl ether, 4-chlorophenyl glycidyl ether, 4-bromophenyl glycidyl ether, 2,4,6-trichlorophenyl glycidyl ether, 2,4,6-tribromophenyl glycidyl ether, pentafluorophenyl glycidyl ether, o-cresyl glycidyl ether, m-cresyl glycidyl ether, p-cresyl glycidyl ether, glycidyl acetate, glycidyl cyclohexylcarboxylate, glycidyl benzoate, and *N*-glycidyl phthalimide.

Preferred monoepoxide compounds are ethylene oxide, propylene oxide, 1,2-butene oxide, 2,3-butene oxide, styrene oxide, butyl glycidyl ether, benzyl glycidyl ether, phenyl glycidyl ether, p-tolyl glycidyl ether, 4-tert-butylphenyl glycidyl ether.

A mixture of the aforementioned monoepoxide compounds can also be used.

Diepoxide compounds are for example butadiene diepoxide, vinylcyclohexene diepoxide, limonene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, polybutadiene diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl ethers of polybutadiene - bisphenol-A - block-copolymers, diglycidyl o-phthalate, diglycidyl isophthalate, diglycidyl terephthalate.

Preferred diepoxide compounds are butadiene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl o-phthalate, diglycidyl isophthalate, diglycidyl terephthalate.

A mixture of two or more the aforementioned diepoxides can also be used.

Polyepoxide compounds are for example glycerol polyglycidyl ether, trimethylolpropane polyglycidyl ether, pentaerythritol polyglycidyl ether, diglycerol polyglycidyl ether, polyglycerol polyglycidyl ether, sorbitol polyglycidyl ether.

A mixture of one or more polyepoxide compounds and/or one or more of the aforementioned diepoxide compounds can also be used.

Onium salts within the scope of the invention are salts represented by formula (I)

$$[M(R1)(R2)(R3)(R4)]^{+}_{n}Y^{n-} \qquad (I)$$

wherein

M is phosphorous or antimony,

(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with one or more alkyl groups containing 1 to 10 carbon atoms and/or heteroatom containing substituents and/or heteroatoms,
whereas
(R4) is different from (R1), (R2), and (R3) and
wherein (R4) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and aryl groups, containing 6 carbon atoms which are substituted with a heteroatom containing group,whereas
(R1), (R2) are aryl groups containing 6 to 18 carbon atoms preferably 6 carbon atoms,
Y is a halide, carbonate, nitrate, sulphate or phosphate anion and
n is an integer of 1, 2 or 3.

In one embodiment of the invention M of formula (I) is phosphorous.

In another embodiment of the invention (R1), (R2), (R3) of formula (I) independently of one another are aryl groups containing 6 to 18 carbon atoms, preferably (R1), (R2), (R3) of formula (I) are each phenyl groups.

In another embodiment of the invention (R4) of formula (I) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and phenyl, substituted with at least one heteroatom containing group, wherein the heteroatom is selected from O, N and/or S.

In another embodiment of the invention (R4) of formula (I) is selected from the group comprising of branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and phenyl, substituted with a O-methyl, O-ethyl, O-propyl, O-butyl, O-phenyl, N-(methyl)$_2$-, N-(ethyl)$_2$-, N-(phenyl)$_2$, S- methyl, S-ethyl, S-propyl, S-butyl, or S-phenyl- group.

In another embodiment of the invention (R4) of formula (I) is selected from *i*-propyl, *i*-butyl, *sec*-butyl, *t*-butyl, *n*-pentyl, *iso*-pentyl, *neo*-pentyl, *n*-hexyl, 2-hexyl, 3-hexyl, 2-ethyl-hexyl, cyclopentyl, cyclohexyl, cycloheptyl, cyclooctyl, $CH_2$-cyclopentyl, $CH_2$-cyclohexyl, $CH_2$-cycloheptyl, $CH_2$-norbornyl, $CH_2$-bicyclo-[2.2.1]-heptyl, $CH_2$-adamantyl, $CH_2$-bicyclo-[2.2.2]-octyl, $CH_2$-twistyl, $CH_2$-bicyclo-[3.3.3]-undecyl, 2-methoxyphenyl, 4-methoxyphenyl, 2-ethoxyphenyl, 4-ethoxyphenyl, 2-propoxyphenyl, 4-propoxyphenyl, 2-isopropoxyphenyl, 4-isopropoxyphenyl, 2-phenoxyphenyl, 4-phenoxyphenyl, 2-(N,N-dimethylamino)phenyl, 4-(N,N-dimethylamino)phenyl, 2-(N,N-ethylmethylamino)-phenyl, 4-(N,N-ethylmethylamino)-phenyl, 2-(N,N-diethylamino)-phenyl, 4-(N,N-diethylamino)-phenyl, 2-(N-pyrrolidyl)-phenyl, 4-(N-pyrrolidyl)-phenyl, 2-(methylthio)-phenyl, 4-(methylthio)-phenyl, 2-(ethylthio)-phenyl and 4-(ethylthio)-phenyl.

In another preferred embodiment of the invention a catalyst of formula (I) is used, wherein
M is phosphorous, (R1), (R2) and (R3) are each phenyl groups and
(R4) is selected from the group comprising *i*-propyl, *i*-butyl, *sec*-butyl, *t*-butyl, *n*-pentyl, *iso*-pentyl, *neo*-pentyl, *n*-hexyl, 2-hexyl, 3-hexyl, 2-ethyl-hexyl, cyclopentyl, cyclohexyl, cycloheptyl, cyclooctyl, $CH_2$-cyclopentyl, $CH_2$-cyclohexyl, $CH_2$-cycloheptyl, $CH_2$-norbornyl, $CH_2$-bicyclo-[2.2.1]-heptyl, $CH_2$-adamantyl, $CH_2$-bicyclo-[2.2.2]-octyl, $CH_2$-twistyl, $CH_2$-bicyclo-[3.3.3]-undecyl, 2-methoxyphenyl, 4-methoxyphenyl, 2-ethoxyphenyl, 4-ethoxyphenyl, 2-propoxyphenyl, 4-propoxyphenyl, 2-isopropoxyphenyl, 4-isopropoxyphenyl, 2-phenoxyphenyl, 4-phenoxyphenyl, 2-(N,N-dimethylamino)phenyl, 4-(N,N-dimethylamino)phenyl, 2-(N,N-ethylmethylamino)-phenyl, 4-(N,N-ethylmethylamino)-phenyl, 2-(N,N-diethylamino)-phenyl, 4-(N,N-diethylamino)-phenyl, 2-(N-pyrrolidyl)-phenyl, 4-(N-pyrrolidyl)-phenyl, 2-(methylthio)-phenyl, 4-(methylthio)-phenyl, 2-(ethylthio)-phenyl and 4-(ethylthio)-phenyl,
Y is a halide, carbonate, nitrate, sulphate or phosphate anion, preferably a halide or carbonate anion, more preferred chloride, bromide or carbonate and
n is an integer of 1, 2 or 3.

In another embodiment of the invention a catalyst of formula (I) is used, wherein
M is phosphorous, (R1), (R2) and (R3) are each phenyl groups and
(R4) is selected from the group comprising cyclopentyl, cyclohexyl, $CH_2$-adamantyl, 2-methoxyphenyl and 4-methoxyphenyl,
Y is a halide, carbonate, nitrate, sulphate or phosphate anion, preferably a halide or carbonate anion, more preferred chloride, bromide or carbonate and
n is an integer of 1, 2 or 3.

In another embodiment of the invention in combination with one or more of the aforementioned embodiments the isocyanate compound is selected from benzyl isocyanate, phenyl isocyanate, *ortho-, meta-, para-tolyl* isocyanate,

dimethylphenyl isocyanate (technical mixture and individual isomers), 4-cyclohexylphenyl isocyanate and/or *ortho-, meta-, para*-methoxyphenyl isocyanate and the epoxide compound is selected from ethylene oxide, propylene oxide, 1,2-butene oxide, 2,3-butene oxide, styrene oxide, butyl glycidyl ether, benzyl glycidyl ether, phenyl glycidyl ether, p-tolyl glycidyl ether and/or 4-tert-butylphenyl glycidyl ether.

In another embodiment of the invention in combination with one or more of the aforementioned embodiments the diisocyanate compound is selected from hexamethylene diisocyanate (HDI), 3-isocyanatomethyl-3,3,5-trimethylcyclohexyl isocyanate (isophorone diisocyanate, IPDI), 4,4'-diisocyanatodicyclohexylmethane ($H_{12}$-MDI), 2,4- or 2,5- or 2,6-diisocyanatotoluene (TDI) or mixtures of these isomers and/or 4,4'-, 2,4'- or 2,2'-diisocyanatodiphenylmethane (MDI) or mixtures of these isomers and the diepoxide compound is selected from butadiene diepoxide, the diepoxides of double unsaturated fatty acid C1 - C18 alkyl esters, ethylene glycol diglycidyl ether, di(ethylene glycol) diglycidyl ether, poly(ethylene glycol) diglycidyl ether, propylene glycol diglycidyl ether, di(propylene glycol) diglycidyl ether, poly(propylene glycol) diglycidyl ether, neopentyl glycol diglycidyl ether, 1,6-hexanediol diglycidyl ether, hydrogenated bisphenol-A diglycidyl ether, 1,2-dihydroxybenzene diglycidyl ether, resorcinol diglycidyl ether, 1,4-dihydroxybenzene diglycidyl ether, bisphenol-A diglycidyl ether, diglycidyl o-phthalate, diglycidyl isophthalate and/or diglycidyl terephthalate.

In one embodiment the method according to the invention is performed in the presence of a solvent. Suitable solvents are high-boiling non-protic solvents such as *N*-methylpyrrolidone (NMP), *N*-ethylpyrrolidone, cyclic ethylene carbonate, cyclic propylene carbonate, sulfolane, chlorobenzene, the different isomers of dichlorobenzene, the different isomers of trichlorobenzene, decalin, hexamethylphosphoramide, dimethylformamide, *N,N*-dimethylacetamide (DMAc), dimethyl sulfoxide or mixtures of one or more of the aforementioned solvents among each other or with further non-protic solvents. Preferred solvents are NMP, sulfolane and DMAc.

In another embodiment of the method according to the invention, the reaction is conducted in the absence of a solvent. Preferably, the reaction mixture contains only the epoxide compound(s), the isocyanate compound(s) and the onium salt(s) as well as the oxazolidinone compound formed during the reaction.

The isocyanate compound may be added to the epoxide compound in a continuous or step-wise manner with two or more individual addition steps in the step-wise addition wherein in each individual addition step the amount of isocyanate compound added is ≤ 50 weight-% of the total amount of isocyanate compound to be added. This is to be understood in such a way that during the course of the reaction the isocyanate compound is added to the reaction mixture containing the epoxide compound continuously or in the aforementioned step-wise manner. Included is also the case that the isocyanate compound is added *via* a syringe pump, dripping funnel or other continuous or semi-continuous devices where the isocyanate is brought into the reaction mixture. Although some after-reaction time may be given to the reaction system, the reaction should be essentially complete shortly after the end of the addition of the isocyanate compound.

By way of process criteria, one could establish a condition that 30 minutes, preferably 20 minutes and more preferred 10 minutes after the end of the isocyanate addition no more change in the NCO group content of the reaction mixture takes place (within the boundaries of experimental uncertainty). This may be observed, for example, by *in-situ* IR spectroscopy or analysis of samples of the reaction mixture concerning their NCO content, for example, by titration according to DIN ISO 10283.

In one embodiment of the method according to the invention, the isocyanate compound is added continuously to the reaction mixture. "Continuously" in the meaning of the invention means that the isocyanate compound is added to the reaction mixture over a defined period of time, while at the same time any isocyanate compound present in the reaction mixture is converted to the oxazolidinone compound. Preferably, the rate of isocyanate addition is smaller than or equal to the maximum rate, with which the isocyanate compound can be converted under these reaction conditions to the oxazolidinone compound in order to avoid accumulation of NCO groups in the reaction mixture. The actual concentration of NCO groups in the reaction mixture may be observed, for example, by *in-situ* IR spectroscopy. If the NCO group concentration is observed to increase above a set value, the rate of isocyanate addition is reduced. Preferably, the isocyanate compound is added to the reaction mixture (consisting of epoxide compound, isocyanate compound, onium salt and oxazolidinone compound, but not considering solvent, if present) with such an addition rate that the concentration of the isocyanate compound in the reaction mixture is ≤ 40 weight-%, preferably ≤ 20 weight-% and more preferred ≤ 15 weight-%.

In another embodiment of the method according to the invention, the amount of isocyanate compound added in each individual addition step is ≥ 0.1 weight-% to ≤ 50 weight-% of the total amount of isocyanate compound to be added. Preferably, the amount of isocyanate compound added per individual addition step is ≥ 1.0 weight-% to ≤ 40 weight-%, more preferred ≥ 5.0 weight-% to ≤ 35 weight-% of the total amount of isocyanate compound to be added. Preferably, the time intervals between each individual addition of isocyanate compound to the reaction mixture (consisting of epoxide compound, isocyanate compound, onium salt and oxazolidinone compound, but not considering solvent, if present) is chosen in such a way that the concentration of the isocyanate compound in the reaction mixture at any given point in time is ≤ 40 weight-%, preferably ≤ 20 weight-% and more preferred ≤ 15 weight-%. The actual concentration of NCO groups in the reaction mixture may be observed, for example, by *in-situ* IR spectroscopy. If the NCO group

concentration is observed to increase above a set value, the time interval between the addition steps is increased.

In another embodiment of the method according to the invention, the isocyanate compound is an isocyanate compound with two or more NCO groups per molecule, preferably with two NCO groups per molecule, and the epoxide compound is an epoxide compound with two or more epoxy groups per molecule, preferably with two epoxide groups per molecule.

The method according to the invention is performed for example at temperatures of ≥ 130 °C to ≤ 280 °C, preferably at a temperature of ≥ 140 °C to ≤ 240 °C, most preferred at a temperature of ≥ 155 °C to ≤ 210 °C.

In another embodiment of the invention the catalyst according to formula (I) is present in an amount of ≥ 0.0001 mol-% to ≤ 2.0 mol-%, relative to the amount of isocyanate compound to be added to the reaction mixture. Preferably, the catalyst is present in an amount of ≥ 0.001 mol-% to ≤ 1.5 mol-%, more preferred ≥ 0.01 mol-% to ≤ 1.1 mol-%.

In another embodiment of the method according to the invention, the oxazolidinone product is obtained with a chemoselectivity $S_{OXA}$, given as the ratio of the molar amount of oxazolidinone moieties to the sum of the molar amount of oxazolidinone moieties and the molar amount of isocyanurate groups, of > 88 %, preferably ≥ 95 % and particularly preferred ≥ 99 %. The molar ratio can be determined, for example, using high performance liquid chromatography (HPLC) or NMR spectroscopy. One method for determining the chemoselectivity $S_{OXA}$ is described below in the methods.

In another embodiment of the method according to the invention the oxazolidinone product is obtained with a regioselectivity towards the 5-substituted 1,3-oxazolidin-2-one regioisomer of ≥ 78 %, preferably ≥ 80 % and particularly preferred ≥ 88 %. The regioselectivity $R_{5\text{-}OXA}$ is given as the ratio of the molar fraction of 5-substituted 1,3-oxazolidin-2-one moieties to the sum of the molar fractions of 5-substituted and 4-substituted 1,3-oxazolidin-2-one moieties. The molar fraction of the 5-substituted and 4-substituted 1,3-oxazolidin-2-one regioisomers can be determined, for example, using high performance liquid chromatography (HPLC) or NMR spectroscopy. One method for determining the regioselectivity $R_{5\text{-}OXA}$ is described below in the methods.

In another embodiment of the method according to the invention compounds according to the general formula (I) are used for the manufacture of oligomeric or polymeric oxazolidinone compounds with a regioselectivity towards the 5-substituted 1,3-oxazolidin-2-one regioisomer of ≥ 78 %.

$$[M(R1)(R2)(R3)(R4)]^{+}{}_{n}Y^{n-} \qquad (I)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with one or more alkyl groups containing 1 to 10 carbon atoms and/or heteroatom containing substituents and/or heteroatoms,
whereas
(R4) is different from (R1), (R2), and (R3) and
is selected from the group comprising branched alkyl groups containing 3 to 22 carbon atoms, cycloaliphatic groups containing 3 to 22 carbon atoms, C1 to C3 alkyl-bridged cycloaliphatic groups containing 3 to 22 carbon atoms and aryl groups, containing 6 to 18 carbon atoms, optionally substituted with one or more alkyl groups containing 1 to 10 carbon atoms and/or heteroatom containing substituents and/or heteroatoms,
whereas
(R1), (R2) are aryl groups containing 6 to 18 carbon atoms,
Y is a halide, carbonate, nitrate, sulfate or phosphate anion and
n is an integer of 1, 2 or 3.

The present invention is further directed towards an oligomeric or polymeric oxazolidinone compound, obtainable by a method according to the invention using an isocyanate compound with at least two NCO groups per molecule, preferably two NCO groups per molecule, and an epoxide compound with at least two epoxy groups per molecule, preferably two epoxy groups per molecule, comprising at least one unit derived from the isocyanate compound and at least two units derived from the epoxide compound or comprising at least one unit derived from the epoxide compound and at least two units derived from the isocyanate compound.

The chain length of the polyoxazolidinone compounds can be controlled by adjusting the ratio between the diisocyanate and the diepoxide compound. An isocyanate terminated oligomer is obtained, when the diisocyanate is employed in excess. An epoxide terminated oligomer is obtained, when the diepoxide is employed in excess. Linear

polymer chains with high molecular weight are obtained, when equimolar amounts of diisocyanate and diepoxide are reacted with one another. The precise content of isocyanate and epoxide groups in the diisocyanate and diepoxide, respectively, are preferentially determined before the polymerisation reaction, *e.g.,* by measuring the isocyanate number according to German standard norm DIN EN ISO 11909 and the epoxide number according to German standard norm DIN EN 1877-1.

The number average molecular weight $M_n$ of the polyoxazolidinone compound is in the range of ≥ 2’000 g/mol to ≤ 200’000 g/mol, as determined with gel permeation chromatography (GPC). Another method suitable to determine the molecular weight of the polyoxazolidinone compounds is based on end-group analysis with $^1$H-NMR spectroscopy.

An alternative method to control the molecular weight of the products constitutes in adding a small amount of a monoepoxide or monoisocyanate to the mixture of polyepoxide and the polyisocyanate compound. The monoepoxide or monoisocyanate compound can be added at the beginning of the reaction of polyepoxide and polyisocyanate compound, during the reaction of polyepoxide and polyisocyanate compound or after the reaction of polyepoxide and polyisocyanate compound is completed.

Preferably this oligomeric or polymeric oxazolidinone compound comprises at least one, preferably at least two and particularly preferred two terminal epoxide and/or isocyanate groups.

It is also possible that this oligomeric or polymeric oxazolidinone compound comprises at least one terminal group which is non-reactive towards epoxide and/or isocyanate groups. Examples include alkoxy groups or trialkylsiloxane groups.

The method according to the invention is suited for the synthesis of oxazolidinone compounds with interesting properties for use, for example, as pharmaceutics or antimicrobiotics. Polyoxazolidinones obtained by the method according to the invention are particularly suited as polymer building blocks in polyurethane chemistry. For example, epoxy-terminated oligomeric oxazolidinones (oligooxazolidinones) may be reacted with polyols or polyamines to form foams or thermosets. Such epoxy-terminated oligomeric oxazolidinones are also suited for the preparation of composite materials. Epoxy-terminated oligomeric oxazolidinones (oligooxazolidinones) may also be reacted with their NCO-terminated counterparts to form high molecular weight polyoxazolidinones, which are useful as transparent, high temperature-stable materials. Polyoxazolidinones with high molecular weight obtained by the method according to the invention are particularly suited as transparent, high temperature-stable thermoplastic materials.

## Examples

*Isocyanates*

*para-Tolyl* isocyanate, purity 99%; distilled in a partial vacuum (200 mbar, 80 °C) and stored under argon at -4 °C prior to use, Acros Organics, Belgium.
Diisocyanate 1: 2,4-Toluenediisoyanate, >99 % 2,4-isomer, Bayer MaterialScience AG, Germany

*Epoxides*

*R*-(+)-Styrene oxide was distilled in a partial vacuum (200 mbar, 80 °C) prior to use. 1,2-Epoxy-3-phenoxy propane; distilled in a partial vacuum (200 mbar) prior to use.
Diepoxide 1: Bisphenol-A-diglycidylether (4,4’-isopropylidenediphenol diglycidyl ether, Epikote™ Resin 162), purity +99%, stored at room temperature, Momentive, Germany

*Phosphonium salts*

| | |
|---|---|
| I-1: $[PPh_3\{p\text{-}C_6H_4(OMe)\}]Br$ | Synthesised as specified in the examples below |
| I-2: $PPh_3\{p\text{-}C_6H_4(OMe)\}]_2(CO_3)$ | Synthesised as specified in the examples below |
| 1-3: $[PPh_4]Br$ | Purchased from Sigma-Aldrich, Germany |
| I-4: $[P(n\text{-}Bu)_4]Br$ | Purchased from Sigma-Aldrich, Germany |
| I-5: $[PPh_3(CH_2\text{-adamantyl})]Br$ | Purchased from Sigma-Aldrich, Germany |
| I-6: $[PPh_3(cyclohexyl)]Br$ | Purchased from Sigma-Aldrich, Germany |

*Solvents*

If not mentioned otherwise, solvents were purchased from Sigma-Aldrich, Germany and used as received.

*N*-Methylpyrrolidinone (NMP), purity 99.5%, anhydrous was obtained from Sigma-Aldrich, Germany.

*Reactor*

The 300 ml pressurized reactor used in the synthesis of polyoxazolidinones had a height (internal) of 10.16 cm and an internal diameter of 6.35 cm. The reactor was fitted with an electric heating jacket (510 watt maximum heating capacity). The counter cooling consisted of a U-shaped dip tube with an external diameter of 6 mm, which projected into the reactor to within 5 mm of the bottom and through which cooling water at approximately 10°C was passed. The water stream was switched on and off by means of a solenoid valve. The reactor was also fitted with an inlet tube and a temperature probe of 1.6 mm diameter, both of which projected into the reactor to within 3 mm of the bottom. The impeller agitator used in the examples was a pitched-blade turbine to which a total of two agitator stages, each with four agitator blades (45°) of 35 mm diameter and 10 mm height, were attached to the agitator shaft at a distance of 7 mm.

In the terminology used in the examples the term "pulse addition", corresponding to the step-wise addition mode of the isocyanate, means that the isocyanate compound is added in individual steps, comprising a part of the total amount of the isocyanate compound, to the reaction mixture.

*In-situ Infrared (IR) spectroscopy*

The composition of the reaction mixtures was followed with a Bruker MATRIX-MF spectrometer equipped with a high-pressure attenuated total reflectance (ATR) IR fibre optical probe. The ATR IR fibre optical probe (3.17 mm outer diameter, 90° diamond prism with 1 $\times$ 2 mm basal area and 1 mm height as ATR element, 2 $\times$ 45° reflection of the IR beam, IR beam coupled *via* fibre optics) was fitted into the reactor in such a way that the diamond at the end of the optical probe was immersed entirely into the reaction mixture. IR spectra (average of 20 scans, if not stated otherwise) were recorded time-resolved in the region of 3500-650 $cm^{-1}$ with a resolution of 4 $cm^{-1}$ at 60 second time intervals (if not stated otherwise).

The spectra were analysed with the software PEAXACT 3.1 - Software for Quantitative Spectroscopy and Chromatography, S•PACT GmbH using the Integrated Hard Model (IHM) method. The hard model for analysing the IR spectra of the reaction mixtures was created by linear combination of hard models for the single components monoisocyanate, oxazolidinone and isocyanurate. The hard model for the single components was created as follows:

Monoisocyanate 1:

A 10 mL glass flask containing 5 mL propylene carbonate was immersed into an oil bath preheated to 160 °C. A background IR spectrum (average of 100 scans) was measured against air. The IR probe was then inserted into the glass flask in such a way that the diamond at the end of the optical probe was immersed entirely into the reaction mixture. After flushing the glass flask with argon, the glass flask was capped with a rubber seal and another background IR spectrum was measured (average of 100 scans). Subsequently, monoisocyanate 1 (0.37 mL, 2.95 mmol, 5 mol% relative to propylene carbonate) was injected with a microliter syringe through the seal into the reaction mixture and several IR spectra were recorded of the resulting mixture. The relevant signals in the region 2150 to 2400 $cm^{-1}$ were selected from a representative IR spectrum and the model refined to achieve an optimum fit between a superposition of the selected signals and the averaged IR spectra of the mixture.

Oxazolidinone 1:

A 10 mL glass flask containing 0.5 mL propylene carbonate was immersed into an oil bath preheated to 160 °C. A background IR spectrum (average of 100 scans) was measured against air. The IR probe was then inserted into the glass flask in such a way that the diamond at the end of the optical probe was immersed entirely into the reaction mixture. After flushing the glass flask with argon, the glass flask was capped with a rubber seal and another background IR spectrum was measured (average of 100 scans). Subsequently, a solution of oxazolidinone 1 (0.471 g, 1.18 mmol, 10 mol% relative to propylene carbonate) in 0.5 mL propylene carbonate was injected with a microliter syringe through the seal into the reaction mixture and several IR spectra were recorded of the resulting mixture. The relevant signals in the region 1650 to 1800 $cm^{-1}$ were selected from a representative IR spectrum and the model refined to achieve an optimum fit between a superposition of the selected signals and the averaged IR spectra of the mixture.

Isocyanurate 1:

A 10 mL glass flask containing 0.5 mL propylene carbonate was immersed into an oil bath preheated to 160 °C. A background IR spectrum (average of 100 scans) was measured against air. The IR probe was then inserted into

the Schlenk flask in such a way that the diamond at the end of the optical probe was immersed entirely into the reaction mixture. After flushing the glass flask with argon, the Glass flask was capped with a rubber seal and another background IR spectrum was measured (average of 100 scans). Subsequently, a solution of isocyanurate 1 (0.471 g, 1.18 mmol, 10 mol% relative to propylene carbonate) in 0.5 mL propylene carbonate was injected with a microliter syringe through the seal into the reaction mixture and several IR spectra were recorded of the resulting mixture. The relevant signals in the region 1600 to 1750 $cm^{-1}$ were selected from a representative IR spectrum and the model refined to achieve an optimum fit between a superposition of the selected signals and the averaged IR spectra of the mixture.

The *in-situ* IR spectra obtained during the catalyst tests were analysed by performing a hard modelling factor analysis (HMFA) as described in E. Kriesten, D. Mayer, F. Alsmeyer, C. B. Minnich, L. Greiner and W. Marquardt, Chemomet. Intell. Lab. Sys., 93(2), 108 (2008) using the hard models obtained by the method described above. The molar amount $n_i$ of the respective component i was calculated.

The turnover frequency ($TOF_{1/2}$) was determined according to equation 1,

$$TOF_{1/2} = \frac{n_{NCO,t_0} - n_{NCO,t_{1/2}}}{n_{cat} \times t_{1/2}} \qquad \text{(eq. 1)}$$

where $t_{1/2}$ is half of the time interval from injection of the second isocyanate pulse until no further decrease of the isocyanate concentration was observed, $n_{NCO,t0}$ is the molar amount of isocyanate in the reaction mixture after injection of the second isocyanate pulse, $n_{NCO,t1/2}$ is the residual molar amount of isocyanate observed by *in-situ* IR spectroscopy at $t_{1/2}$ after injection of the second isocyanate pulse, and $n_{cat}$ is the molar amount of catalyst in the reaction mixture after injection of the second isocyanate pulse.

The chemoselectivity towards oxazolidinone ($S_{OXA}$) was calculated according to equation 2,

$$S_{oxa} = \frac{n^{fin}{}_{OXA}}{n^{fin}{}_{OXA} + n^{fin}{}_{ICT}} \qquad \text{(eq. 2)}$$

where $n^{fin}{}_{OXA}$ is the molar amount of oxazolidinone moieties at the end of the reaction and $n^{fin}{}_{ICT}$ is the molar amount of isocyanurate groups at the end of the reaction.

*Analysis of the product mixture*

The products were analysed with IR spectroscopy, HPLC analysis and NMR spectroscopy as detailed below.

Solid state IR analyses were performed on a Bruker ALPHA-P IR spectrometer equipped with a diamond probe head. A background spectrum was recorded against ambient air. Thereafter, a small sample of the oxazolidinone (2 milligrams) was applied to the diamond probe and the IR spectrum recorded averaging over 32 spectra obtained in the range of 4000 to 400 $cm^{-1}$ with a resolution of 4 $cm^{-1}$. The software OPUS 6.5 was used for data treatment.

In the reaction of isocyanate compounds with epoxide compounds, two different regioisomers the 5-substituted 1,3-oxazolidin-2-one (5-OXA, formula (IIa)) and the 4-substituted 1,3-oxazolidin-2-one (4-OXA, formula (IIb)) are formed, as represented below by the left and right formula, respectively.

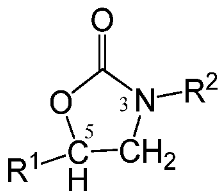

5-OXA

(IIa)

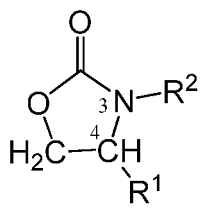

4-OXA

(IIb)

The molar fraction of the two regioisomers in the oxazolidinone product was determined by HPLC chromatography (monooxazolidinones) or $^1H$ NMR spectroscopy (polyoxazolidinones) as described below. The regioselectivity $R_{5\text{-}OXA}$ was then calculated according to the following equation 3:

$$R_{5-OXA} = \frac{x_{5-OXA}}{x_{5-OXA} + x_{4-OXA}} \quad \text{(eq. 3)}$$

where $x_{5\text{-}OXA}$ is the molar fraction of 5-substituted 1,3-oxazolidin-2-one (5-OXA) moieties at the end of the reaction and $x_{4\text{-}OXA}$ is the molar fraction of 4-substituted 1,3-oxazolidin-2-one (4-OXA) moieties at the end of the reaction.
$^1$H NMR spectroscopy: A sample of the reaction mixture or the polyoxazolidinone was dissolved in deuterated dimethyl sulfoxide or $CDCl_3$ and the NMR spectrum recorded on a Bruker AV400 (400 MHz) or Bruker AV600 (600 MHz) instrument. Chemical shifts $\delta$ (given in parts per million, ppm) are reported relative to the residual proton signal of the solvent (dmso-$d^6$, $\delta$ = 2.50 ppm, or $CDCl_3$, $\delta$ = 7.14 ppm).

The HPLC analyses were carried out on an Agilent Technologies 1200 Series instrument equipped with an UV detector and a Waters SymmetryShield $RP_{18}$ column (particle size 5 μm, pore size 100 Å). The temperature of the column was kept constant at 40 °C throughout the measurement. A water-methanol solvent system (flow rate of 1.0 mL/min) was used for separation of the components of the reaction mixture. The reaction mixture (10 mg) and mesitylene (5 μl) were dissolved in methanol (1 ml) and a defined volume of the mixture injected onto the column. An isocratic condition with a water-to-methanol ratio of 60:40 was maintained for 20 min. Then, the methanol content in the eluent was increased steadily to 100 % over another 20 min. The signals for the 4-OXA and 5-OXA compound as well as the isocyanurate were identified by comparing the retention time against pure reference compounds.

*Polymerisation reactions*

The average chain length of the polyoxazolidinones was adjusted by the relative molar ratio of the diisocyanate and diepoxide used in the particular reaction.
When an epoxide terminated polyoxazolidinone is desired, the diepoxide is used in excess. Equation 4 (eq.4) below gives a general mathematical formula to calculate the average chain length n in the oligomeric and/or polymeric product based on the molar ratios of diisocyanate and diepoxide compound employed.

$$n = 1 / [\{(\text{amount of diepoxide in mol}) / (\text{amount of diisocyanate in mol})\} - 1] \quad \text{(eq. 4)}$$

When an isocyanate terminated polyoxazolidinone is desired, the diisocyanate is used in excess. Equation 5 (eq. 5) below gives a general mathematical formula to calculate the average chain length n in the oligomeric and/or polymeric product based on the molar ratios of diisocyanate and diepoxide compound employed.

$$n = 1 / [\{(\text{amount of isocyanate in mol}) / (\text{amount of epoxide in mol})\} - 1] \quad \text{(eq. 5)}$$

The ratio of the two regioisomers in the polyoxazolidinone product was determined by $^1$H NMR spectroscopy. A sample of the oligomer or polymer was dissolved in deuterated dimethyl sulfoxide and the NMR spectrum recorded on a Bruker spectrometer (AV400, 400 MHz). Both regioisomers were identified by the characteristic proton signals in the $^1$H NMR spectra associated with the methine and methylene protons of the heterocyclic oxazolidinone ring fragments.

*System 1: Product of the reaction of 2,4-toluenediisocyanate (TDI) with 4,4'-isopropylidenediphenol diglycidyl ether (BADGE)*

A representative example of the polyoxazolidinone structure is represented by the formula (III) shown below. The formula weight (FW) of the end-groups and the repeating unit is given, based on which the average molecular weight and chain length was estimated.

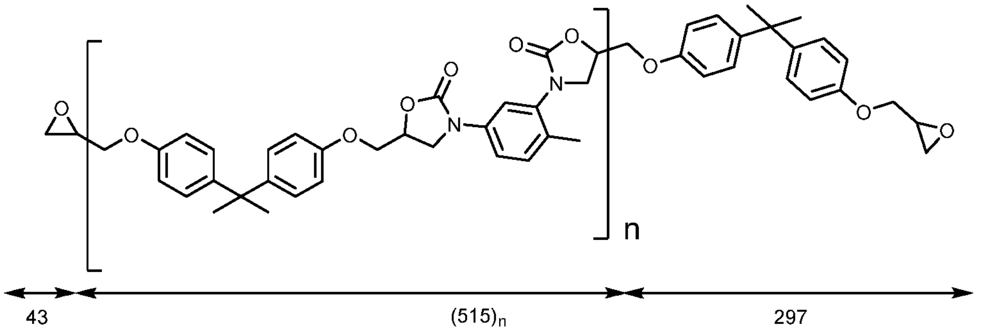

(III)

The relevant resonances in the $^1$H NMR spectra (relative to TMS = 0 ppm), which were used for integration, are as follows:

S1-1: 4.65 ppm; methine of the oxazolidinone ring of 4-OXA, signal references to 1 proton
S1-2: 4.87 ppm; methine of the oxazolidinone ring of 5-OXA, signal references to 1 proton

The regioselectivity $R_{5\text{-OXA}}$ was calculated from the integrals of the signals S1-1 and S1-2 using the following equation 6:

$$R_{5-OXA} = \frac{\text{Integral S1 - 2}}{\text{Integral S1 - 1} + \text{Integral S1 - 2}} \qquad \text{(eq. 6)}$$

Gel permeation chromatography (GPC) measurements were performed at 40 °C in N,N-dimethylacetamide (DMAc, flow rate of 0.6 mL min$^{-1}$). The column set consisted of 4 consecutive columns (GRAM 3000, HEMA300, HEMA 40, HEMA 40). Samples (concentration 10-15 g L$^{-1}$, injection volume 100 μL) were injected employing an HP1200 auto sampler. A UV-detector was used for following the concentration at the exit of the column. Raw data were processed using the PSS WinGPC Unity software package. Polystyrene of known molecular weight was used as reference to calculate the molecular weight distribution. The number average molecular weight measured by GPC is denominated as $M_n$(GPC) in the examples.

*Analysis of the polyoxazolidinone product*

The stability of the polyoxazolidinones was characterised by thermogravimetric analysis (TGA). The measurements were performed on a Mettler Toledo TGA/DSC 1. The sample (6 to 20 mg) was heated from 25 °C to 600 °C with a heating rate of 10 K/min and the relative weight loss followed in dependence of temperature. For data analysis the software STAR$^e$ SW 11.00 was used. As decomposition temperature the inflection point of the sinuidal weight loss curve is stated.

The glass transition point was recorded on a Mettler Toledo DSC 1. The sample (4 to 10 mg) was heated from 25°C to 250 °C with a heating rate of 10 K/min. For data analysis the software STAR$^e$ SW 11.00 was used. For determination of the glass transition temperature a tangential analysis method was used. The midpoint of the intersection point between the tangent at low temperature and the tangent in the mid temperature range and the intersection point between the tangent in the mid temperature range and the tangent at high temperature is stated.

*Synthesis of phosphonium salts*

1-1: *Synthesis of [$PPh_3${p-$C_6H_4$(OMe)}]Br*

A glass flask (20 mL) was charged with 4-bromoanisol (381.5 mg, 2.04 mmol), $PPh_3$ (562 mg, 2.14 mmol), $NiBr_2$ (22.3 mg, 0.10 mmol) and ethylene glycol (0.68 mL, 12.19 mmol). The mixture was stirred (600 rpm) at room temperature for 10 min using a magnetic stirrer bar of 0.3 cm length. The flask was then placed into a hot oil bath set to 180 °C for 20 minutes. The heating of the oil bath was then switched off and the reaction mixture cooled to room temperature within of 30 min. Dichloromethane (35 mL) was added to the crude reaction mixture and the organic layer was washed three times with water and once with brine, dried over $MgSO_4$, filtered and the solvent partially removed under partial vacuum. A brown highly viscous fluid was obtained, which was diluted with dichloromethane (ca. 2 mL). Diethylether (15 mL) was added to this mixture, while shaking the flask vigorously. A white precipitate was obtained,

which was collected, washed two times with diethylether and dried in a partial vacuum to afford the product as a white solid.

1-2: Synthesis of *[PPh$_3${p-C$_6$H$_4$(OMe)}]$_2$(CO$_3$)*

In a glass flask silver carbonate ($Ag_2CO_3$, 0.5 g, 1.81 mmol) was dissolved in MeOH (100 mL) and stirred for 1 h at room temperature under darkness. The clear solution was then added drop-wise to a solution of [PPh$_3${*p*-C$_6$H$_4$(OMe)}]Br (1.5206 g, 3.62 mmol) in MeOH (4 mL) and stirred for 3 h under darkness. The mixture was then left standing overnight. The yellow precipitate was filtered off the next day. The volatiles were removed from the filtrate using a rotary evaporator. A white solid was obtained. The analytical data confirmed that the desired compound [PPh$_3${*p*-C$_6$H$_4$(OMe)}]$_2$(CO$_3$) had been obtained.

Example 1: *Reaction of R-(+)-styrene oxide with para-tolyl isocyanate using [PPh$_3${p-C$_6$H$_4$(OMe)}]Br (I-1) as catalyst*

Under a continuous flow of Argon (2.0 L/h), a three necked glass Schlenk flask (30 mL) was charged with catalyst 1-1 (39.8 mg, 0.0877 mmol) and *R*-(+)-styrene oxide (1.054 g, 8.77 mmol). The mixture was stirred (300 rpm) at room temperature for 10 min using a magnetic stir bar of 0.2 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 45 min. The chemical composition of the reaction mixture was analysed.

$TOF_{1/2}$ with respect to conversion of the isocyanate was 2000 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone compound was 100 %. Signals due to the presence of other side products like carbodiimids and homo-polymers of the epoxides were not observed.

The regioselectivity $R_{5\text{-}OXA}$ (HPLC) towards the 5-oxazolidinone (5-OXA) regioisomer was 88 %.

Example 2: *Reaction of R-(+)-styrene oxide with para-tolyl isocyanate using* [PPh$_3$(CH$_2$-adamantyl)]Br *(I-5) as catalyst*

Under a continuous flow of Argon (2.0 L/h), a glass flask (20 mL) was charged with catalyst 1-5 (43.19 mg, 0.0877 mmol) and *R*-(+)-styrene oxide (1.054 g, 8.77 mmol). The mixture was stirred (800 rpm) at room temperature for 10 min using a magnetic stirrer bar of 0.5 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 30 min. The chemical composition of the reaction mixture was analysed. $TOF_{1/2}$ with respect to conversion of the isocyanate was 580 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone compound was 100 %. Signals due to the presence of other side products like carbodiimides and homo-polymers of the epoxides were not observed.

The regioselectivity $R_{5\text{-}OXA}$ (HPLC) towards the 5-oxazolidinone regioisomer was 78 %.

Example 3: *Reaction of R-(+)-styrene oxide with para-tolyl isocyanate using [PPh$_3$(cyclohexyl)]Br (I-6) as catalyst*

Under a continuous flow of Argon (2.0 L/h), a glass flask (20 mL) was charged with catalyst 1-6 (37.31 mg, 0.0877 mmol) and *R*-(+)-styrene oxide (1.054 g, 8.77 mmol). The mixture was stirred (800 rpm) at room temperature for 10 min using a magnetic stirrer bar of 0.5 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by

disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 30 min. The chemical composition of the reaction mixture was analysed.

$TOF_{1/2}$ with respect to conversion of the isocyanate was 762 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone was 100 %. Signals due to the presence of other side products like carbodiimides and homo-polymers of the epoxides were not observed.

The regioselectivity $R_{5\text{-}OXA}$ (HPLC) towards the 5-oxazolidinone regioisomer was 80 %.

Example 4 (comparative): *Reaction of R-(+)-styrene oxide with para-tolyl isocyanate using $PPh_4Br$ (I-3) as catalyst*

Under a continuous flow of Argon (2.0 L/h), a glass flask (20 mL) was charged with catalyst 1-3 (36.77 mg, 0.0877 mmol) and *R*-(+)-styrene oxide (1.054 g, 8.77 mmol). The mixture was stirred (800 rpm) at room temperature for 10 min using a magnetic stirrer bar of 0.5 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 30 min. The chemical composition of the reaction mixture was analysed.

$TOF_{1/2}$ with respect to conversion of the isocyanate was 552 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone was 100 %. Signals due to the presence of other side products like carbodiimides and homo-polymers of the epoxides were not observed.

The regioselectivity $R_{5\text{-}OXA}$ (HPLC) towards the 5-oxazolidinone regioisomer was < 78 %.

Example 5 (comparative): *Reaction of R-(+)-styrene oxide with para-tolyl isocyanate using* $[P(n\text{-}Bu)_4]Br$ *(I-4) as catalyst*

Under a continuous flow of Argon (2.0 L/h), a glass flask (20 mL) was charged with catalyst 1-4 (29.75 mg, 0.0877 mmol) and *R*-(+)-styrene oxide (1.054 g, 8.77 mmol). The mixture was stirred (800 rpm) at room temperature for 10 min using a magnetic stirrer bar of 0.5 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 30 min. The chemical composition of the reaction mixture was analysed.

$TOF_{1/2}$ with respect to conversion of the isocyanate was 410 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone was 100 %. Signals due to the presence of other side products like carbodiimides and homo-polymers of the epoxides were not observed.

The regioselectivity $R_{5\text{-}OXA}$ (HPLC) towards the 5-oxazolidinone regioisomer was 74 %.

Table 1: Comparison of the results of Examples 1 to 5

| Example | Catalyst | $TOF_{1/2}$ [$h^{-1}$] | Regioselectivity $R_{5\text{-}OXA}$ |
|---|---|---|---|
| 1 | $[PPh_3\{p\text{-}C_6H_4(OMe)\}]Br$ *(I-1)* | 2000 | 88 % |
| 2 | $[PPh_3(CH_2\text{-adamantyl})]Br$ *(I-5)* | 580 | 78 % |
| 3 | $[PPh_3(\text{cyclohexyl})]Br$ *(I-6)* | 762 | 80 % |
| 4 (comp.) | $PPh_4Br$ *(I-3)* | 552 | < 78 % |

(continued)

| Example | Catalyst | $TOF_{1/2}$ [$h^{-1}$] | Regioselectivity $R_{5\text{-}OXA}$ |
|---|---|---|---|
| 5 (comp.) | $[P(n\text{-}Bu)_4]Br$ *(I-4)* | 410 | 74 % |
| (comp.) = comparative example | | | |

Examples 1 to 3 show that the oxazolidinone product is obtained with a higher rate ($TOF_{1/2}$), when phosphonium salts with an unsymmetric substitution pattern are used, in comparison to comparative examples 4 and 5, in which a phosphonium salt with a symmetric substitution pattern was used. The regioselectivities to the 5-oxazolidinone regioisomer obtained in examples 1 to 3 using onium salts according to the invention are equal or higher compared to examples 4 to 5.

Example 6: *Reaction of 1,2-epoxy-3-phenoxy propane with para-tolyl isocyanate using I-1 as catalyst*

Under a continuous flow of argon (2.0 L/h), a glass flask (20 mL) was charged with catalyst 1-1 (39.37 mg, 0.0877 mmol) and 1,2-epoxy-3-phenoxy propane (1.317 g, 8.77 mmol). The mixture was stirred (800 rpm) at room temperature for 10 min using a magnetic stirrer bar of 0.5 cm length. The flask was then placed into a hot oil bath set to 160 °C. The IR probe was immersed into the reaction mixture and the IR spectrometer started. A first pulse of *para-tolyl* isocyanate (0.388 g, 2.92 mmol) was added quickly (within of 2 s) and the reaction mixture stirred until completion of the reaction was indicated by disappearance of the isocyanate band. A second pulse of *para-tolyl* isocyanate (0.388 g, 2.91 mmol) was added quickly (within of 2 s) and the reaction mixture stirred until completion of the reaction was indicated by disappearance of the isocyanate band. A third pulse of *para-tolyl* isocyanate (0.388 g, 2.92 mmol) was added quickly (within of 2 s) and the reaction mixture stirred until completion of the reaction was indicated by disappearance of the isocyanate band. The heating of the oil bath was switched off and the reaction mixture cooled to room temperature within of 30 min. The chemical composition of the reaction mixture was analysed.

$TOF_{1/2}$ with respect to conversion of the isocyanate was 1955 $h^{-1}$.

HPLC analysis confirmed full conversion of 1,2-epoxy-3-phenoxy propane and *para-tolyl* isocyanate.

The chemoselectivity $S_{OXA}$ (HPLC) for the formation of the oxazolidinone was 100 %. Signals due to the presence of other side products like carbodiimides and homo-polymers of the epoxides were not observed.

The regioselectivity $R_{5\text{-}OXA}$ (HPLC) towards the 5-oxazolidinone regioisomer was 100 %.

Table 2: Comparison of the results of examples 1 and 6 using *$[PPh_3\{p\text{-}C_6H_4(OMe)\}]Br$ (I-1)* as catalyst

| Example | Epoxide | $TOF_{1/2}$ [$h^{-1}$] | Regioselectivity $R_{5\text{-}OXA}$ |
|---|---|---|---|
| 1 | *R*-(+)-styrene oxide | 2000 | 88 % |
| 6 | 1,2-epoxy-3-phenoxy propane | 1955 | 100% |

Examples 1 and 6 show that independent of the choice of the epoxide a similar rate is obtained.

Example 7: *Reaction of R-(+)-styrene oxide with para-tolyl isocyanate using $[PPh_3(cyclohexyl)]Br$ (I-6) as catalyst at a higher reaction temperature*

Example 3 was repeated as described above, whereby the reaction was conducted at a temperature of 200 °C.

$TOF_{1/2}$ with respect to conversion of the isocyanate was 812 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone was 95%. Signals due to the presence of other side products like carbodiimides and homo-polymers of the epoxides were not observed.

The regioselectivity $R_{5\text{-}OXA}$ (HPLC) towards the 5-oxazolidinone regioisomer was 85%.

Example 8 (comparative): *Reaction of R-(+)-styrene oxide with para-tolyl isocyanate using $PPh_4Br$ (I-3) as catalyst at a higher reaction temperature*

Example 4 (comp.) was repeated as described above, whereby the reaction was conducted at a temperature of 200 °C.

$TOF_{1/2}$ with respect to conversion of the isocyanate was 661 $h^{-1}$.

HPLC analysis confirmed full conversion of *R*-(+)-styrene oxide and *para-tolyl* isocyanate.

The chemoselectivity $S_{OXA}$ (HPLC) for the formation of the oxazolidinone was 88 %. Signals due to the presence of other side products like carbodiimides and homo-polymers of the epoxides were not observed.
The regioselectivity $R_{5\text{-}OXA}$ (HPLC) towards the 5-oxazolidinone regioisomer was 75 %.

Table 3: Results of the reaction of an isocyanate with an epoxide at a temperature of 200 °C

| Example | Catalyst | Temp. [°C] | $TOF_{1/2}$ [$h^{-1}$] | Chemoselectivity $S_{OXA}$ | Regioselectivity $R_{5\text{-}OXA}$ |
|---|---|---|---|---|---|
| 3 | [$PPh_3$(cyclohexyl)]Br *(I-6)* | 160 | 762 | 100 % | 80 % |
| 7 | [$PPh_3$(cyclohexyl)]Br *(I-6)* | 200 | 812 | 95 % | 85 % |
| 4 (comp.) | $PPh_4Br$ *(I-3)* | 160 | 552 | 100 % | < 78 % |
| 8 (comp.) | $PPh_4Br$ *(I-3)* | 200 | 661 | 88 % | 75 % |
| Comp. = comparative example | | | | | |

Examples 3 and 7 show that independent of the reaction temperature the oxazolidinone product is obtained with a higher rate ($TOF_{1/2}$), when phosphonium salts with an unsymmetric substitution pattern are used, in comparison to comparative examples 4 and 8, in which a phosphonium salt with a symmetric substitution pattern was used. At both reaction temperatures, the regioselectivity towards the 5-oxazolidinone regioisomer obtained in examples 3 to 7 using onium salts according to the invention were higher compared to comparative examples 4 and 8.

Example 9: *Reaction of 2,4-toluenediisocyanate (TDI) with 4,4'-isopropylidenediphenol diglycidyl ether (BADGE) using $PPh_3\{p\text{-}C_6H_4(OMe)\}]_2Br$ (I-1) as catalyst*

The reaction was performed in a 300 ml stainless steel autoclave as described above with a motor with a stirring power of 90 W. The reactor was charged with diepoxide 1 (BADGE, 15.0 g, 44.1 mmol) and catalyst 1-2 (154 mg, 0.42 mmol) and subsequently flushed three times with argon. After addition of dry NMP (40 ml), the reactor was sealed and the mixture was heated to 200 °C under rapid stirring (1000 rpm). After 200 °C had been obtained, a solution of diisocyanate 1 (TDI, 7.3 g, 41.97 mmol) in dry NMP (15 ml) was added with an HPLC pump (10 ml pump head) over 3 h. After an overall reaction time of 19 h (starting with the addition of diisocyanate 1), the reaction mixture was cooled to room temperature. The reaction mixture was poured into methanol. The precipitate was collected and washed three times with methanol and subsequently with diethylether. The obtained powder was dried at $5\times10^{-2}$ mbar for 4 hours.
The presence of oxazolidinone moieties was confirmed by the characteristic IR-signal at v=1749 $cm^{-1}$. The IR spectra did not show the characteristic isocyanurate-band in the region of 1690-1710 $cm^{-1}$. Therefore, the chemoselectivity $S_{OXA}$ towards oxazolidinone was > 96%.
The regioselectivity $R_{5\text{-}OXA}$ (NMR) towards the 5-substituted 1,3-oxazolidin-2-one regioisomer was ≥ 96 %.

$M_n$(GPC): 6660 g/mol
$T_D$: 413 °C
$T_g$: 148 °C

Example 9 shows that polyoxazolidinone compounds are obtained, when onium salts according to the invention are used as catalyst in the reaction of diisocyanate compounds with diepoxide compounds.

Example 10: *Reaction of 2,4-toluenediisocyanate (TDI) with 4,4'-isopropylidenediphenol diglycidyl ether (BADGE) using $PPh_3\{p\text{-}C_6H_4(OMe)\}]_2Br$ (I-1) as catalyst followed by reaction of the product with para-tolyl isocyanate*

The reaction was performed in a 300 ml stainless steel autoclave as described above with a motor with a stirring power of 90 W. The reactor was charged with diepoxide 1 (15.0 g, 44.1 mmol) and catalyst 1-1 (0.2g, 0.44 mmol) and subsequently flushed with Argon. After addition of dry NMP (40 ml), the reactor was sealed and the mixture was heated to 200 °C under rapid stirring (1000 rpm). After 200 °C had been obtained, a solution of diisocyanate 1 (7.3 g, 41.97 mmol) in dry NMP (15 ml) was added with an HPLC pump (10 ml pump head) over 3 h. After the diisocyanate solution had been completely added, *para*-tolyl-isocyanate (0.23 g, 1.7 mmol) dissolved in dry NMP (20 ml) was added. After an overall reaction time of 19 h (starting with addition of diisocyanate 1), the reaction mixture was cooled down to room temperature. The reaction mixture was poured into methanol. The precipitate was collected and washed three times with methanol and subsequently with diethylether. The obtained powder was dried at $5\times10^{-2}$ mbar for 4 hours.
The presence of oxazolidinone moieties was confirmed by the characteristic IR-signal at v=1749 $cm^{-1}$. The IR

spectra did not show the characteristic isocyanurate-band in the region of 1690-1710 $cm^{-1}$. Therefore, the chemoselectivity $S_{OXA}$ towards oxazolidinone was > 96%.

The regioselectivity $R_{5\text{-}OXA}$ (NMR) towards the 5-substituted 1,3-oxazolidin-2-one regioisomer was ≥ 95 %.

$M_n$(GPC) : 6930 g/mol
$T_D$: 420 °C
$T_g$: 149 °C

Example 10 shows that polyoxazolidinone compounds are obtained, when onium salts according to the invention are used as catalyst in the reaction of diisocyanate compounds with diepoxide compounds followed by reaction of the obtained product with a monoisocyanate compound in the presence of the same catalyst.

## Claims

1. A method for the production of oxazolidinone compounds, comprising the step of reacting an isocyanate compound with an epoxide compound in the presence of a catalyst, **characterized in that** the catalyst is represented by the general formula (I)

$$[M(R1)(R2)(R3)(R4)]^{+}nY^{n-} \quad (I)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with one or more alkyl groups containing 1 to 10 carbon atoms and/or heteroatom containing substituents and/or heteroatoms,
whereas
(R4) is different from (R1), (R2), and (R3) and
wherein (R4) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and aryl groups, containing 6 carbon atoms which are substituted with a heteroatom containing group,
whereas
(R1), (R2) are aryl groups containing 6 to 18 carbon atoms,
Y is a halide, carbonate, nitrate, sulfate or phosphate anion and
n is an integer of 1, 2 or 3.

2. A method according to claim 1, wherein M is phosphorous.

3. A method according to claim 1 or 2, wherein (R1), (R2), (R3) independently of one another are aryl groups comprising 6 to 18 carbon atoms.

4. A method according to one or more of claims 1 to 3, wherein (R1), (R2) and (R3) are each phenyl groups.

5. A method according to one or more of claims 1 to 4, wherein (R4) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and phenyl, substituted with at least one heteroatom containing group, wherein the heteroatom is selected from O, N and/or S.

6. A method according to one or more of claims 1 to 5, wherein (R4) is selected from the group comprising branched alkyl groups containing 3 to 6 carbon atoms, cycloaliphatic groups containing 3 to 8 carbon atoms, C1 to C3 alkyl-bridged cycloaliphatic groups containing 3 to 12 carbon atoms and phenyl, substituted with a O-methyl, O-ethyl, O-propyl, O-butyl, O-phenyl, N-$(methyl)_2$-, N-$(ethyl)_2$-, N-$(phenyl)_2$, S- methyl, S-ethyl, S-propyl, S-butyl or S-phenyl-

group.

**7.** A method according to one or more of claims 1 to 6, wherein (R4) is selected from *i*-propyl, *i*-butyl, *sec*-butyl, *t*-butyl, *n*-pentyl, *iso*-pentyl, *neo*-pentyl, *n*-hexyl, 2-hexyl, 3-hexyl, 2-ethyl-hexyl, cyclopentyl, cyclohexyl, cycloheptyl, cyclooctyl, $CH_2$-cyclopentyl, $CH_2$-cyclohexyl, $CH_2$-cycloheptyl, $CH_2$-norbornyl, $CH_2$-bicyclo-[2.2.1]-heptyl, $CH_2$-adamantyl, $CH_2$-bicyclo-[2.2.2]-octyl, $CH_2$-twistyl, $CH_2$-bicyclo-[3.3.3]-undecyl, 2-methoxyphenyl, 4-methoxyphenyl, 2-ethoxyphenyl, 4-ethoxyphenyl, 2-propoxyphenyl, 4-propoxyphenyl, 2-isopropoxyphenyl, 4-isopropoxyphenyl, 2-phenoxyphenyl, 4-phenoxyphenyl, 2-(N,N-dimethylamino)phenyl, 4-(N,N-dimethylamino)phenyl, 2-(N,N-ethylmethylamino)-phenyl, 4-(N,N-ethylmethylamino)-phenyl, 2-(N,N-diethylamino)-phenyl, 4-(N,N-diethylamino)-phenyl, 2-(N-pyrrolidyl)-phenyl, 4-(N-pyrrolidyl)-phenyl, 2-(methylthio)-phenyl, 4-(methylthio)-phenyl, 2-(ethylthio)-phenyl and 4-(ethylthio)-phenyl.

**8.** Method according to one or more of claims 1 to 7 wherein the isocyanate compound is added to the epoxide compound in a continuous or step-wise manner with two or more individual addition steps in the step-wise addition, wherein in each individual addition step the amount of isocyanate compound added is $\leq$ 50 weight-% of the total amount of isocyanate compound to be added.

**9.** Method according to one or more of claims 1 to 8 wherein the reaction is conducted at a temperature of $\geq$ 130 °C to $\leq$ 280 °C.

**10.** An oxazolidinone compound, obtainable by a method according to one or more of claims 1 to 9, with a regioselectivity towards the 5-substituted 1,3-oxazolidin-2-one regioisomer between $\geq$ 78 % and $\leq$ 100 %, and wherein the number average molecular weight $M_n$ of the polyoxazolidinone compound is in the range of $\geq$ 2'000 g/mol to $\leq$ 200'000 g/mol, as determined with gel permeation chromatography (GPC).

**11.** An oligomeric or polymeric oxazolidinone compound, obtainable by a method according to one or more of claims 1 to 9 using an isocyanate compound with two NCO groups per molecule and an epoxide compound with two epoxy groups per molecule, comprising at least one unit derived from the isocyanate compound and at least two units derived from the epoxide compound, and wherein the number average molecular weight $M_n$ of the polyoxazolidinone compound is in the range of $\geq$ 2'000 g/mol to $\leq$ 200'000 g/mol, as determined with gel permeation chromatography (GPC).

**12.** The compound according to claim 11, comprising at least one terminal epoxide and/or isocyanate group or comprising at least one terminal group which is non-reactive towards epoxide and/or isocyanate groups.

**13.** Use of compounds according to the general formula (I)

$$[M(R1)(R2)(R3)(R4)]^{+}{}_{n}Y^{n-} \qquad (I)$$

wherein

M is phosphorous or antimony,
(R1), (R2), (R3), (R4) are independently of one another selected from the group comprising linear or branched alkyl groups containing 1 to 22 carbon atoms, optionally substituted with heteroatoms and/or heteroatom containing substituents, cycloaliphatic groups containing 3 to 22 carbon atoms, optionally substituted with heteroatoms and/or heteroatom containing substituents, C1 to C3 alkyl-bridged cycloaliphatic groups containing 3 to 22 carbon atoms, optionally substituted with heteroatoms and/or heteroatom containing substituents and aryl groups containing 6 to 18 carbon atoms, optionally substituted with one or more alkyl groups containing 1 to 10 carbon atoms and/or heteroatom containing substituents and/or heteroatoms,
whereas
(R4) is different from (R1), (R2), and (R3) and
is selected from the group comprising branched alkyl groups containing 3 to 22 carbon atoms, cycloaliphatic groups containing 3 to 22 carbon atoms, C1 to C3 alkyl-bridged cycloaliphatic groups containing 3 to 22 carbon atoms and aryl groups, containing 6 to 18 carbon atoms, optionally substituted with one or more alkyl groups containing 1 to 10 carbon atoms and/or heteroatom containing substituents and/or heteroatoms,
whereas
(R1), (R2) are aryl groups containing 6 to 18 carbon atoms,
Y is a halide, carbonate, nitrate, sulfate or phosphate anion and

n is an integer of 1, 2 or 3.

for the manufacture of oligomeric or polymeric oxazolidinone compounds with a regioselectivity towards the 5-substituted 1,3-oxazolidin-2-one regioisomer of ≥ 78 %.

**Patentansprüche**

1. Verfahren zur Herstellung von Oxazolidinonverbindungen, umfassend den Schritt der Umsetzung einer Isocyanatverbindung mit einer Epoxidverbindung in Gegenwart eines Katalysators,
**dadurch gekennzeichnet, dass** der Katalysator durch die allgemeine Formel (I) wiedergegeben wird

$$[M(R1)(R2)(R3)(R4)]^{+}_{n}Y^{n-} \qquad (I),$$

wobei

M für Phosphor oder Antimon steht,
(R1), (R2), (R3), (R4) unabhängig voneinander aus der geradkettige oder verzweigte Alkylgruppen mit 1 bis 22 Kohlenstoffatomen, gegebenenfalls substituiert durch Heteroatome und/oder heteroatomhaltige Substituenten, cycloaliphatische Gruppen mit 3 bis 22 Kohlenstoffatomen, gegebenenfalls substituiert durch Heteroatome und/oder heteroatomhaltige Substituenten, C1- bis C3-alkylverbrückte cycloaliphatische Gruppen mit 3 bis 22 Kohlenstoffatomen, gegebenenfalls substituiert durch Heteroatome und/oder heteroatomhaltige Substituenten, und Arylgruppen mit 6 bis 18 Kohlenstoffatomen, gegebenenfalls substituiert durch eine oder mehrere Alkylgruppen mit 1 bis 10 Kohlenstoffatomen und/oder heteroatomhaltigen Substituenten und/oder Heteroatomen, umfassenden Gruppe ausgewählt sind,
wobei
(R4) von (R1), (R2) und (R3) verschieden ist und wobei (R4) aus der verzweigte Alkylgruppen mit 3 bis 6 Kohlenstoffatomen, cycloaliphatische Gruppen mit 3 bis 8 Kohlenstoffatomen, C1- bis C3-alkylverbrückten cycloaliphatischen Gruppen mit 3 bis 12 Kohlenstoffatomen und Arylgruppen mit 6 Kohlenstoffatomen, die durch eine heteroatomhaltige Gruppe substituiert sind, umfassenden Gruppe ausgewählt sind,
wobei
(R1), (R2) für Arylgruppen mit 6 bis 18 Kohlenstoffatomen stehen,
Y für ein Halogenid-, Carbonat-, Nitrat-, Sulfat- oder Phosphatanion steht und
n für eine ganze Zahl 1, 2 oder 3 steht.

2. Verfahren nach Anspruch 1, wobei M für Phosphor steht.

3. Verfahren nach Anspruch 1 oder 2, wobei (R1), (R2), (R3) unabhängig voneinander für Arylgruppen mit 6 bis 18 Kohlenstoffatomen stehen.

4. Verfahren nach einem oder mehreren der Ansprüche 1 bis 3, wobei (R1), (R2) und (R3) jeweils für Phenylgruppen stehen.

5. Verfahren nach einem oder mehreren der Ansprüche 1 bis 4, wobei (R4) aus der verzweigte Alkylgruppen mit 3 bis 6 Kohlenstoffatomen, cycloaliphatische Gruppen mit 3 bis 8 Kohlenstoffatomen, C1- bis C3-alkylverbrückte cycloaliphatische Gruppen mit 3 bis 12 Kohlenstoffatomen und Phenyl, substituiert durch mindestens eine heteroatomhaltige Gruppe, wobei das Heteroatom aus O, N und/oder S ausgewählt ist, umfassenden Gruppe ausgewählt sind.

6. Verfahren nach einem oder mehreren der Ansprüche 1 bis 5, wobei (R4) aus der verzweigte Alkylgruppen mit 3 bis 6 Kohlenstoffatomen, cycloaliphatische Gruppen mit 3 bis 8 Kohlenstoffatomen, C1- bis C3-alkylverbrückten cycloaliphatischen Gruppen mit 3 bis 12 Kohlenstoffatomen und Phenyl, substituiert durch eine O-Methyl-, O-Ethyl-, O-Propyl-, O-Butyl-, O-Phenyl-, N-(Methyl)$_2$-, N-(Ethyl)$_2$-, N-(Phenyl)$_2$-, S-Methyl-, S-Ethyl-, S-Propyl-, S-Butyl- oder S-Phenylgruppe, umfassenden Gruppe ausgewählt sind.

7. Verfahren nach einem oder mehreren der Ansprüche 1 bis 6, wobei (R4) aus Isopropyl, Isobutyl, sek.-Butyl, t-Butyl, n-Pentyl, Isopentyl, Neopentyl, n-Hexyl, 2-Hexyl, 3-Hexyl, 2-Ethylhexyl, Cyclopentyl, Cyclohexyl, Cycloheptyl, Cyclooctyl, $CH_2$-Cyclopentyl, $CH_2$-Cyclohexyl, $CH_2$-Cycloheptyl, $CH_2$-Norbornyl, $CH_2$-Bicyclo[2.2.1]heptyl, $CH_2$-Adamantyl, $CH_2$-Bicyclo[2.2.2]octyl, $CH_2$-Twistyl, $CH_2$-Bicyclo[3.3.3]undecyl, 2-Methoxyphenyl, 4-Methoxyphenyl, 2-Ethoxyphenyl, 4-Ethoxyphenyl, 2-Propoxyphenyl, 4-Propoxyphenyl, 2-Isopropoxyphenyl, 4-Isopropoxyphenyl, 2-Phenoxyphenyl, 4-Phenoxyphenyl, 2-(N,N-Dimethylamino)phenyl, 4-(N,N-Dimethylamino)phenyl, 2-(N,N-Ethylmethylamino)phenyl, 4-(N,N-Ethylmethlyamino)phenyl, 2-(N,N-Diethylamino)-phenyl, 4-(N,N-Diethylamino)phenyl, 2-(N-Pyrrolidyl)phenyl, 4-(N-Pyrrolidyl)phenyl, 2-(Methylthio)phenyl, 4-(Methylthio)phenyl, 2-(Ethylthio)phenyl und 4-(Ethylthio)phenyl ausgewählt ist.

**8.** Verfahren nach einem oder mehreren der Ansprüche 1 bis 7, wobei die Isocyanatverbindung kontinuierlich oder schrittweise, mit zwei oder mehr einzelnen Zugabeschritten bei der schrittweisen Zugabe, zu der Epoxidverbindung gegeben wird,
wobei bei jedem einzelnen Zugabeschritt die Menge an zugegebener Isocyanatverbindung $\leq$ 50 Gew.-% der Gesamtmenge der zuzugebenden Isocynatverbindung beträgt.

**9.** Verfahren nach einem oder mehreren der Ansprüche 1 bis 8, wobei die Umsetzung bei einer Temperatur von $\geq$ 130°C bis $\leq$ 280°C durchgeführt wird.

**10.** Oxazolidinonverbindung, erhältlich nach einem Verfahren nach einem oder mehreren der Ansprüche 1 bis 9, mit einer Regioselektivität für das 5-substituierte 1,3-Oxazolidin-2-on-Regioisomer von $\geq$ 78% bis $\leq$ 100%, wobei das zahlenmittlere Molekulargewicht $M_n$ der Polyoxazolidinonverbindung im Bereich von $\geq$ 2 000 g/mol bis $\leq$ 200 000 g/mol, bestimmt durch Gelpermeationschromatografie (GPC) liegt.

**11.** Oligomere oder polymere Oxazolidinonverbindung, erhältlich nach einem Verfahren nach einem oder mehreren der Ansprüche 1 bis 9 unter Verwendung einer Isocyanatverbindung mit zwei NCO-Gruppen pro Molekül und einer Epoxidverbindung mit zwei Epoxygruppen pro Molekül, umfassend mindestens eine von der Isocyanatverbindung stammende Einheit und mindestens zwei von der Epoxidverbindung stammende Einheiten, wobei das zahlenmittlere Molekulargewicht $M_n$ der Polyoxazolidinonverbindung im Bereich von $\geq$ 2 000 g/mol bis $\leq$ 200 000 g/mol, bestimmt durch Gelpermeationschromatografie (GPC), liegt.

**12.** Verbindung nach Anspruch 11, umfassend mindestens eine endständige Epoxid- und/oder Isocyanatgruppe oder umfassend mindestens eine endständige Gruppe, die nicht gegenüber Epoxid- und/oder Isocyanatgruppen reaktiv ist.

**13.** Verwendung von Verbindungen der allgemeinen Formel (I)

$$[M(R1)(R2)(R3)(R4)]^{+}{}_{n}Y^{n-} \qquad (I),$$

wobei

M für Phosphor oder Antimon steht,
(R1), (R2), (R3), (R4) unabhängig voneinander aus der geradkettige oder verzweigte Alkylgruppen mit 1 bis 22 Kohlenstoffatomen, gegebenenfalls substituiert durch Heteroatome und/oder heteroatomhaltige Substituenten, cycloaliphatische Gruppen mit 3 bis 22 Kohlenstoffatomen, gegebenenfalls substituiert durch Heteroatome und/oder heteroatomhaltige Substituenten, C1- bis C3-alkylverbrückte cycloaliphatische Gruppen mit 3 bis 22 Kohlenstoffatomen, gegebenenfalls substituiert durch Heteroatome und/oder heteroatomhaltige Substituenten, und Arylgruppen mit 6 bis 18 Kohlenstoffatomen, gegebenenfalls substituiert durch eine oder mehrere Alkylgruppen mit 1 bis 10 Kohlenstoffatomen und/oder heteroatomhaltigen Substituenten und/oder Heteroatomen, umfassenden Gruppe ausgewählt sind,
wobei
(R4) von (R1), (R2) und (R3) verschieden ist und aus der verzweigte Alkylgruppen mit 3 bis 22 Kohlenstoffatomen, cycloaliphatische Gruppen mit 3 bis 22 Kohlenstoffatomen, C1- bis C3-alkylverbrückte cycloaliphatische Gruppen mit 3 bis 22 Kohlenstoffatomen und Arylgruppen mit 6 bis 18 Kohlenstoffatomen, gegebenenfalls substituiert durch eine oder mehrere Alkylgruppen mit 1 bis 10 Kohlenstoffatomen und/oder heteroatomhaltige Substituenten und/oder Heteroatome, umfassenden Gruppe ausgewählt ist,
wobei
(R1), (R2) für Arylgruppen mit 6 bis 18 Kohlenstoffatomen stehen,
Y für ein Halogenid-, Carbonat-, Nitrat-, Sulfat- oder Phosphatanion steht und
n für eine ganze Zahl 1, 2 oder 3 steht,

zur Herstellung von oligomeren oder polymeren Oxazolidinonverbindungen mit einer Regioselektivität für das 5-substituierte 1,3-Oxazolidin-2-on-Regioisomer von ≥ 78%.

## Revendications

**1.** Procédé pour la production de composés de type oxazolidinone, comprenant l'étape de mise en réaction d'un composé de type isocyanate avec un composé de type époxyde en présence d'un catalyseur, **caractérisé en ce que** le catalyseur est représenté par la formule générale (I)

$$[M(R1)(R2)(R3)(R4)]^{+}_{n}Y^{n-} \qquad (I)$$

M étant phosphore ou antimoine,
(R1), (R2), (R3), (R4) étant indépendamment les uns des autres choisis dans le groupe comprenant des groupes alkyle linéaires ou ramifiés contenant 1 à 22 atome(s) de carbone, éventuellement substitués par des hétéroatomes et/ou par des substituants contenant un hétéroatome, des groupes cycloaliphatiques comprenant 3 à 22 atomes de carbone, éventuellement substitués par des hétéroatomes et/ou par des substituants contenant un hétéroatome, des groupes cycloaliphatiques pontés par $C_{1-3}$-alkyle contenant 3 à 22 atomes de carbone, éventuellement substitués par des hétéroatomes et/ou par des substituants contenant un hétéroatome et des groupes aryle possédant 6 à 18 atomes de carbone, éventuellement substitués par un ou plusieurs groupes alkyle contenant 1 à 10 atome(s) de carbone et/ou substituants contenant un hétéroatome et/ou hétéroatomes, tandis que
(R4) est différent de (R1), (R2), et (R3), et
(R4) étant choisi dans le groupe comprenant des groupes alkyle ramifiés contenant 3 à 6 atomes de carbone, des groupes cycloaliphatiques contenant 3 à 8 atomes de carbone, des groupes cycloaliphatiques pontés par $C_{1-3}$-alkyle contenant 3 à 12 atomes de carbone et des groupes aryle contenant 6 atomes de carbone qui sont substitués par un groupe contenant un hétéroatome,
tandis que
(R1), (R2) sont des groupes aryle contenant 6 à 18 atomes de carbone,
Y est un anion halogénure, carbonate, nitrate, sulfate ou phosphate et
n est un entier parmi 1, 2 et 3.

**2.** Procédé selon la revendication 1, M étant phosphore.

**3.** Procédé selon la revendication 1 ou 2, (R1), (R2), (R3), indépendamment les uns des autres, étant des groupes aryle comprenant 6 à 18 atomes de carbone.

**4.** Procédé selon l'une ou plusieurs des revendications 1 à 3, (R1), (R2), et (R3) étant chacun des groupes phényle.

**5.** Procédé selon l'une ou plusieurs des revendications 1 à 4, (R4) étant choisi dans le groupe comprenant des groupes alkyle ramifiés contenant 3 à 6 atomes de carbone, des groupes cycloaliphatiques contenant 3 à 8 atomes de carbone, des groupes cycloaliphatiques pontés par $C_{1-3}$-alkyle contenant 3 à 12 atomes de carbone et phényle, substitué par au moins un groupe contenant un hétéroatome, l'hétéroatome étant choisi parmi O, N et/ou S.

**6.** Procédé selon l'une ou plusieurs des revendications 1 à 5, (R4) étant choisi dans le groupe comprenant des groupes alkyle ramifiés contenant 3 à 6 atomes de carbone, des groupes cycloaliphatiques contenant 3 à 8 atomes de carbone, des groupes cycloaliphatiques pontés par $C_{1-3}$-alkyle contenant 3 à 12 atomes de carbone et phényle, substitué par un groupe O-méthyle, O-éthyle, O-propyle, O-butyle, O-phényle, N-(méthyle)$_{2-}$, N-(éthyle)$_2$-, N-(phényle)$_2$, S-méthyle, S-éthyle, S-propyle, S-butyle ou S-phényle.

**7.** Procédé selon l'une ou plusieurs des revendications 1 à 6, (R4) étant choisi parmi *i*-propyle, *i*-butyle, *sec*-butyle, *t*-butyle, *n*-pentyle *iso*-pentyle, néo-pentyle, *n*-hexyle, 2-hexyle, 3-hexyle, 2-éthyl-hexyle, cyclopentyle, cyclohexyle, cycloheptyle, cyclooctyle, $CH_2$-cyclopentyle, $CH_2$-cyclohexyle, $CH_2$-cycloheptyle, $CH_2$-norbornyle, $CH_2$-bicyclo-[2.2.1]-heptyle, $CH_2$-adamantyle, $CH_2$-bicyclo-[2.2.2]-octyle, $CH_2$-twistyle, $CH_2$-bicyclo-[3.3.3]-undécyle, 2- méthoxyphényle, 4-méthoxyphényle, 2-éthoxyphényle, 4-éthoxyphényle, 2-propoxyphényle, 4-propoxyphényle, 2-isopropoxyphényle, 4-isopropoxyphényle, 2-phénoxyphényle, 4-phénoxyphényle, 2-(N,N-diméthylamino)phényle, 4-(N,N-diméthylamino)phényle, 2-(N,N-éthylméthylamino)-phényle, 4-(N,N-éthylméthylamino)-phényle, 2-(N,N-diéthylamino)-phényle, 4-(N,N-diéthylamino)-phényle, 2-(N-pyrrolidinyl)-phényle, 4-(N-pyrrolidinyl)-phényle, 2-(méthylthio)-phényle, 4-(méthylthio)-phényle, 2-(éthylthio)-phényle et 4-(éthylthio)-phényle.

**8.** Procédé selon l'une ou plusieurs des revendications 1 à 7, le composé de type isocyanate étant ajouté au composé de type époxyde d'une manière continue ou graduellement, avec deux étapes d'addition individuelles ou plus dans l'addition graduelle,
dans chaque étape d'addition individuelle la quantité de composé de type isocyanate ajoutée étant ≤ 50 % en poids de la quantité totale de composé de type isocyanate devant être ajoutée.

**9.** Procédé selon l'une ou plusieurs des revendications 1 à 8, la réaction étant conduite à une température de ≥ 130°C à ≤ 280 °C.

**10.** Composé de type oxazolidinone, pouvant être obtenu par un procédé selon l'une ou plusieurs des revendications 1 à 9, avec une régiosélectivité pour le régioisomère de 1,3-oxazolidin-2-one substitué en position 5 comprise entre ≥ 78 % et ≤ 100 %, et le poids moléculaire moyen en nombre $M_n$ du composé de type polyoxazolidinone se situant dans la plage allant de ≥ 2 000 g/mole à ≤ 200 000 g/mole, tel que déterminé par chromatographie à perméation de gel (CPG).

**11.** Composé de type oxazolidinone oligomérique ou polymérique, pouvant être obtenu par un procédé selon l'une ou plusieurs des revendications 1 à 9 à l'aide d'un composé de type isocyanate comportant deux groupes NCO par molécule et un composé de type époxyde comportant deux groupes époxy par molécule, comprenant au moins un motif issu du composé de type isocyanate et au moins deux motifs issus du composé de type époxyde, et le poids moléculaire moyen en nombre $M_n$ du composé de type polyoxazolidinone se situant dans la plage allant de ≥ 2 000 g/mole à ≤ 200 000 g/mole, tel que déterminé par chromatographie à perméation de gel (CPG).

**12.** Composé selon la revendication 11, comprenant au moins un groupe terminal époxyde et/ou isocyanate ou comprenant au moins un groupe terminal qui n'est pas réactif envers des groupes époxyde et/ou isocyanate.

**13.** Utilisation de composés selon la formule générale (I)

$$[M(R1)(R2)(R3)(R4)]^{+}_{n}Y^{n-} \qquad (I)$$

M étant phosphore ou antimoine,
(R1), (R2), (R3), (R4) étant indépendamment les uns des autres choisis dans le groupe comprenant des groupes alkyle linéaires ou ramifiés contenant 1 à 22 atome(s) de carbone, éventuellement substitués par des hétéroatomes et/ou par des substituants contenant un hétéroatome, des groupes cycloaliphatiques comprenant 3 à 22 atomes de carbone, éventuellement substitués par des hétéroatomes et/ou par des substituants contenant un hétéroatome, des groupes cycloaliphatiques pontés par $C_{1-3}$-alkyle contenant 3 à 22 atomes de carbone, éventuellement substitués par des hétéroatomes et/ou par des substituants contenant un hétéroatome et des groupes aryle possédant 6 à 18 atomes de carbone, éventuellement substitués par un ou plusieurs groupes alkyle contenant 1 à 10 atome(s) de carbone et/ou substituants contenant un hétéroatome et/ou hétéroatomes, tandis que
(R4) est différent de (R1), (R2), et (R3), et
est choisi dans le groupe comprenant des groupes alkyle ramifiés contenant 3 à 22 atomes de carbone, des groupes cycloaliphatiques contenant 3 à 22 atomes de carbone, des groupes cycloaliphatiques pontés par $C_{1-3}$-alkyle contenant 3 à 22 atomes de carbone et des groupes aryle contenant 6 à 18 atomes de carbone, éventuellement substitués par un ou plusieurs groupes alkyle contenant 1 à 10 atome(s) de carbone et/ou substituants contenant un hétéroatome et/ou hétéroatomes,
tandis que
(R1), (R2) sont des groupes aryle contenant 6 à 18 atomes de carbone,
Y est un anion halogénure, carbonate, nitrate, sulfate ou phosphate et
n est un entier parmi 1, 2 et 3,

pour la préparation de composés de type oxazolidinone oligomériques ou polymériques avec une régiosélectivité pour le régioisomère de 1,3-oxazolidin-2-one substitué en position 5 de ≥ 78 %.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 12192611
- EP 0296450 A
- EP 0343064 A
- US 3687897 A
- WO 8606734 A1


### Non-patent literature cited in the description


- **M. E. DYEN ; D. SWERN.** *Chem. Rev.,* 1967, vol. 67, 197
- **X. ZHANG ; W. CHEN.** *Chem. Lett.,* 2010, vol. 39, 527
- **M.T. BARROS ; A.M.F. PHILLIPS.** *Tetrahedron: Asymmetry,* 2010, vol. 21, 2746
- **H.-Y. WU ; J.-C. DING ; Y.-K. LIU.** *J. Indian Chem. Soc.,* 2003, vol. 80, 36
- **C. QIAN ; D. ZHU.** *Synlett,* 1994, 129
- **E. KRIESTEN ; D. MAYER ; F. ALSMEYER ; C. B. MINNICH ; L. GREINER ; W. MARQUARDT.** *Chemomet. Intell. Lab. Sys.,* 2008, vol. 93 (2), 108